(19) Europäisches Patentamt
European Patent Office
Office européen des brevets

(11) **EP 3 578 976 A1**

## EUROPEAN PATENT APPLICATION
published in accordance with Art. 153(4) EPC

(43) Date of publication:
**11.12.2019 Bulletin 2019/50**

(21) Application number: **18747693.2**

(22) Date of filing: **19.01.2018**

(51) Int Cl.:
**G01N 33/50** (2006.01)   **C12Q 1/68** (2018.01)
**G01N 33/15** (2006.01)   **G01N 33/497** (2006.01)
**G01N 33/53** (2006.01)   **G01N 33/84** (2006.01)
**C07K 14/705** (2006.01)

(86) International application number:
**PCT/JP2018/001504**

(87) International publication number:
**WO 2018/142961 (09.08.2018 Gazette 2018/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **02.02.2017 JP 2017017380**

(71) Applicant: **Kao Corporation**
**Chuo-Ku**
**Tokyo 103-8210 (JP)**

(72) Inventors:
• **YOSHIKAWA, Keiichi**
  **Haga-gun**
  **Tochigi 321-3497 (JP)**
• **TAKATOKU, Hiroko**
  **Haga-gun**
  **Tochigi 321-3497 (JP)**

(74) Representative: **Vossius & Partner**
**Patentanwälte Rechtsanwälte mbB**
**Siebertstrasse 3**
**81675 München (DE)**

(54) **METHOD FOR SELECTING ODOR CONTROL SUBSTANCE**

(57)    Provided is a method for searching a substance which enhances an odor. The method is a method for selecting a substance which causes enhancement or suppression of an odor and includes measuring a response of a broadly tuned olfactory receptor in the presence of a test substance and selecting a test substance which enhances or decreases the response of the olfactory receptor as a substance which causes enhancement or suppression of an odor.

**Description**

Field of the Invention

The present invention relates to a novel method for controlling an odor based on an odor recognition mechanism.

Background of the Invention

A smell plays an important role to spend comfortable daily life. For example, smells add rich flavor to food, and smells imparted to daily necessaries provide various comforts such as cleanliness. At the same time, unpleasant smells are also important. Odors also play a major role in quickly notifying that health and hygiene are being impaired. The importance of smells can be imagined from that the taste of meals is impaired by occurrence of a respiratory disease, such as a cold, and that an elderly person with reduced olfactory sensitivity suffers from danger without being aware of the odor imparted to gas. If a technology that makes a smell more clearly felt can be constructed a sufficient effect can be provided while reducing the amount of the fragrance agent for imparting the smell in order to provide people with an affluent life.

Many of odorants causing smells are volatile low molecular weight organic compounds having a molecular weight of about 30 to 300. These odorants have diversity not only in the quality of odors but also in chemical structures and are often classified based on the chemical structures in studies from the molecular biological standpoint. For example, in Non-Patent Literature 1, 125 odorants are classified into 13 groups consisting of amines, thiols, alcohols, esters, ethers, aldehydes, cyclic alkanes, terpenes, vanillins, camphors, azines, musks, and ketones and others. In addition, in Patent Literature 1, odorants are classified into 9 groups consisting of amines, alcohols, aldehydes, ketones, acids, esters, benzenes, vanillin-like compounds, and lactones.

These odorants are recognized by olfactory receptors possessed by olfactory sensory neurons (OSNs) spreading in the deep part of the nasal cavity. An olfactory receptor is activated by being bound to an odorant and causes an electrical excitation in an olfactory nerve cell. This electrical excitation is transmitted to the higher-order brain through nerve connection to perceive the odor. In a human being, there are about 400 or more genes encoding olfactory receptors. Such a large number of receptors is the molecular basis enabling detection of the above-mentioned various odors. These about 400 olfactory receptors are each expressed in different olfactory sensory neurons (OSNs). The olfactory sensory neurons (OSNs) expressing the same olfactory receptors converge to the same characteristic region in the projection destination of the axon in the olfactory bulb. Accordingly, the information on which olfactory receptor has been activated in the nasal cavity is reflected on the geometric information on the olfactory bulb region of ignition, and is transmitted to the higher-order brain region without being lost. It is believed that information on combination of olfactory receptors which are activated by individual odorants is thus transferred to the higher-order brain region to make a difference in the quality of odors.

Knowledge on the relationship between odor and olfactory receptor involved in recognition thereof has been accumulated. According to them, in general, olfactory receptors selectively recognize structurally similar odorants. For example, Non-Patent Literature 1 suggests that about 45% of about 1000 olfactory receptors of mice recognize only one group among 13 groups of chemical structurally classified odorants. Patent Literature 1 demonstrates that one olfactory receptor of mice, MOR215-1, does not recognize any of odorants classified into the above-mentioned 9 groups and does recognize selectively a macrocyclic ketone structure exhibiting a musk smell. In contrast, Non-Patent Literature 1 suggests that 13.4% of the olfactory receptors recognize the odorants in 5 to 9 groups, in particular, 1.8% of the olfactory receptors recognize the odorants spreading a significantly wide structural range in 10 to 12 groups. These olfactory receptors are described as broadly tuned olfactory receptors. As a typical broadly tuned olfactory receptor, Non-Patent Literature 1 mentions mouse Olfr42. In addition, OR2W1, which is a human homologous gene, is mentioned as a broadly tuned olfactory receptor in Non-Patent Literature 2.

What is the meaning for an organism of having a broadly tuned olfactory receptor which recognizes a wide range of odorants without distinction? Non-Patent Literature 2 proposes the following four hypotheses: The broadly tuned olfactory receptor is present for increasing the types of odorants which can be detected (1), contributes to an increase in the combination pattern of olfactory receptors to be activated by one odorant and helps distinguishing between odors (2), has a role of making aware of the presence of any odor, not of telling the quality of the odor (3), and recognizes the total concentration of odorants and converts it into sensory intensity, not tells the quality of the odor (4). However, currently, the roles of broadly tuned olfactory receptors are unclear.

Patent Literatures 2 to 13 disclose compounds that activate OR2W1. Patent Literatures 2 to 11 disclose the use of some compounds having actions as OR2W1 antagonists for suppressing a variety of offensive odors. However, since these compounds change not only the activity of OR2W1 but also the actions of other olfactory receptors, the mechanism of OR2W1 contributing to the acceptance of the odors of the compounds is unclear.

Citation List

Patent Literature

(Patent Literature 1) International Patent Publication No. WO 2015/020158
(Patent Literature 2) Japanese Patent No. 5798382
(Patent Literature 3) Japanese Patent No. 5593271
(Patent Literature 4) Japanese Patent No. 5982044
(Patent Literature 5) Japanese Patent No. 5646255
(Patent Literature 6) Japanese Patent No. 5697383
(Patent Literature 7) International Patent Publication No. WO 2012/029922
(Patent Literature 8) JP-A-2015-91802
(Patent Literature 9) JP-A-2015-202075
(Patent Literature 10) JP-A-2015-211667
(Patent Literature 11) JP-A-2015-202077
(Patent Literature 12) Japanese Patent No. 5520173
(Patent Literature 13) JP-A-2016-008955

Non-Patent Literature

(Non-Patent Literature 1) Nara, K. et al, J. Neurosci., 31: 9179-91, 2011
(Non-Patent Literature 2) Yu, Y. et al, Proc. Natl. Acad. Sci. USA, 112: 14966-71, 2015

Summary of the Invention

The present invention provides a method for selecting a substance which causes enhancement or suppression of an odor, the method comprising:

measuring a response of a broadly tuned olfactory receptor in the presence of a test substance; and
selecting a test substance which enhances or decreases the response of the olfactory receptor as a substance which causes enhancement or suppression of an odor.

In addition, the present invention provides a method for selecting a substance which causes enhancement or suppression in olfactory sensitivity, the method comprising:

measuring a response of a broadly tuned olfactory receptor in the presence of a test substance; and
selecting a test substance which enhances or decreases the response of the olfactory receptor as a substance which causes enhancement or suppression in olfactory sensitivity.

Brief Description of the Drawings

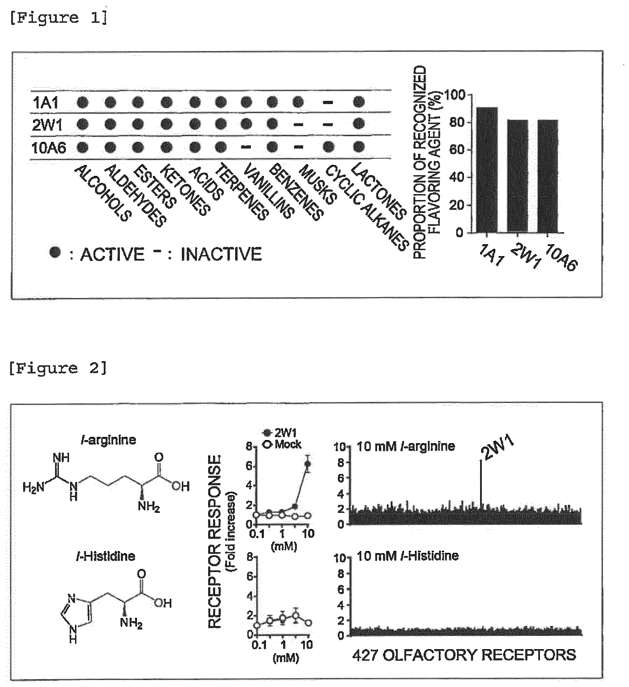

[Figure 1] Figure 1 shows the responsiveness of human olfactory receptors OR2W1, OR1A1, and OR10A6 to substance groups having different chemical structures on the left; and response proportions (%) of OR2W1, OR1A1, and OR10A6 to 11 substance groups on the right.
[Figure 2] Figure 2 shows the response of an olfactory receptor to l-arginine (the top) and l-histidine (the bottom). The left: chemical structures, the middle: responses of OR2W1-expressing cells shown by the averages and standard errors of three independent experiments, where Mock shows the response of cells not expressing OR2W1, and the right: responses of 427 human olfactory receptors.

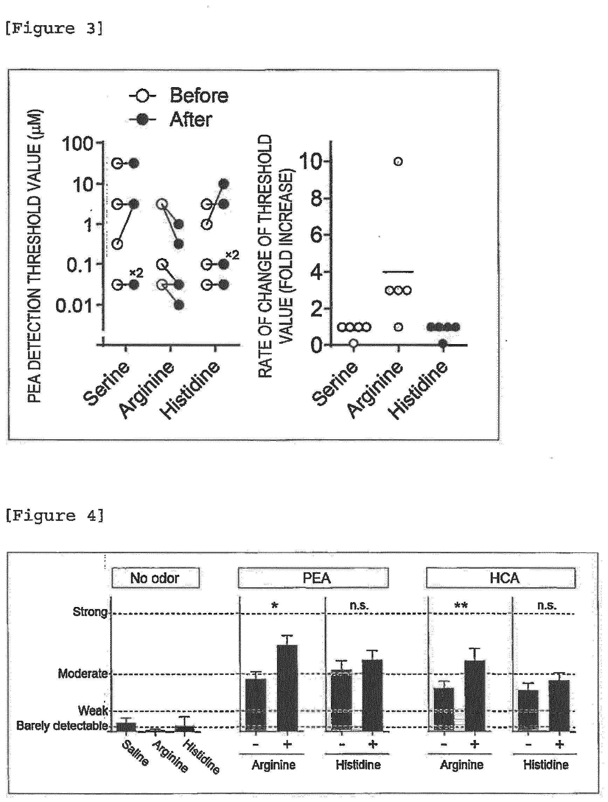

[Figure 3] Figure 3 shows the effect of l-arginine on increasing the detection threshold value of PEA odor. The left: detection threshold values, where two points connected by a solid line in the graph represent PEA detection threshold value concentrations before and after nasal administration of saline, 10 mM 1-arginine in a saline medium, or 10 mM l-histidine in a saline medium in each examinee. In the figure, "×2" represents that there are two examinees showing the data. The right: rates of change of threshold value, where the bar in the graph represents average. *: P < 0.05 (Kruskal-WallisANOVA, and post-test by Dunn test; comparison between saline and 1-arginine or saline and 1-histidine).

[Figure 4] Figure 4 shows the effect of l-arginine on increasing the detection strengths of PEA and HCA odors. The left: evaluation of odor intensity in spraying of saline or a saline aqueous solution of l-arginine or 1-histidine alone. The middle: evaluation of PEA odor intensity in the presence or absence of l-arginine or 1-histidine, where (+): simultaneous use of 1-arginine or l-histidine and PEA, (-): PEA alone. The right: evaluation of HCA odor intensity in the presence or absence of l-arginine or l-histidine, where (+): simultaneous use of 1-arginine or l-histidine and HCA, (-): HCA alone. The data show averages (n = 8 in the graphs on the left and right, n = 10 in the graph in the middle). The error bars are standard errors. *: P < 0.05, **: P < 0.001 (Two-way repeated-measure ANOVA with Sidak's post-hoc test).

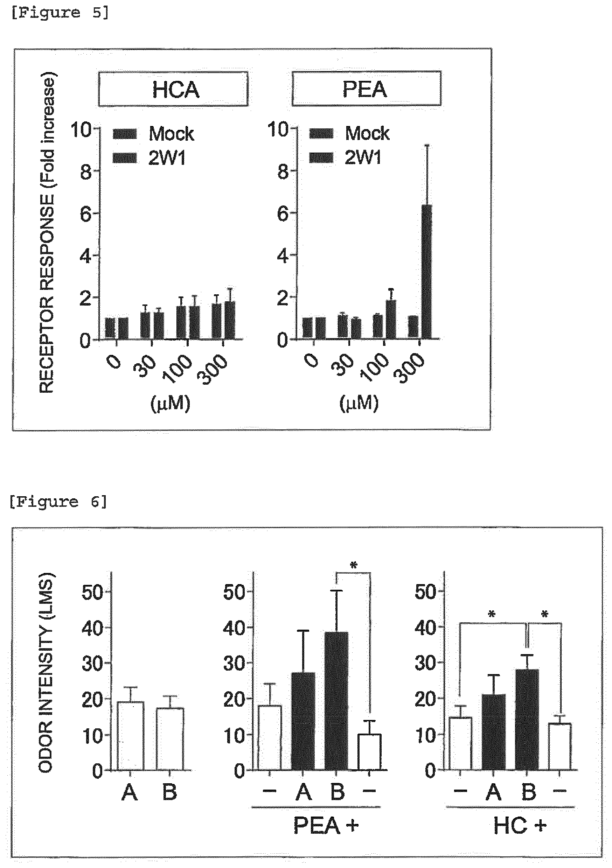

[Figure 5] Figure 5 shows the responsiveness of OR2W1 to PEA and HCA shown by the data of three independent experiments. Only the response of Mock cells to PEA is the data of two independent experiments. All the data are shown by averages and standard errors. Mock shows the response of cells not expressing OR2W1.

[Figure 6] Figure 6 shows the odor enhancing actions of OR2W1 activating substances. The left: odor intensities of compounds A and B, and the middle and the right: effects of enhancing the odors of PEA (middle) and HC (right) by compounds A and B. The bars in each graph represent odor intensities of odorants in, from the left, (-): odorant alone (1st time), (A): odorant + compound A, (B): odorant + compound B, (-): odorant alone (2nd time). The bars and error bars in each graph represent averages and standard errors, respectively, (n = 5). *: P < 0.05, Non-parametric one-way ANOVA (Friedman test) and Dunn's post-test.

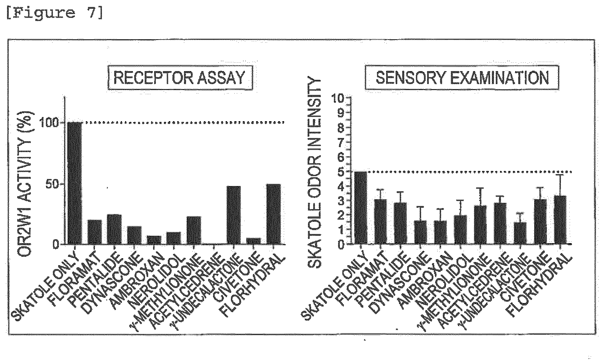

[Figure 7] Figure 7 shows OR2W1 activating actions and skatole odor suppressing actions of OR2W1 antagonist compounds. The horizontal axis of each graph represents OR2W1 antagonist compounds. The vertical axis of the left graph represents the skatole response (odor response (%)) of OR2W1 in the presence of each antagonist compound. The vertical axis of the right graph represents skatole odor suppressing activity of each antagonist in a sensory examination (average of sensory evaluation by 4 to 8 panelists) .

Detailed Description of the Invention

In the present specification, the identity of an amino acid sequence is calculated by Lipman-Pearson method (Science, 1985, 227: 1435-41), specifically, calculated by analysis using a homology analysis (Search homology) program of genetic information processing software Genetyx-Win (Ver. 5.1.1: Software Development) with setting Unit size to compare (ktup) at 2.

In the present specification, the phrase "identity of at least 80%" describing an amino acid sequence refers to an identity of 80% or more, preferably 85% or more, more preferably 90% or more, further preferably 95% or more, further more preferably 98% or more, and more preferably 99% or more.

In the present specification, the term "olfactory receptor polypeptide" refers to an olfactory receptor or a polypeptide having a function equivalent to that of the olfactory receptor; the phrase "polypeptide having a function equivalent to that of an olfactory receptor" refers to a polypeptide which can be expressed on a cell membrane and has a function of increasing the amount of intracellular cAMP and activated by being bound to an odor molecule to activate adenylate cyclase by conjugating with intracellular $G\alpha s$ or $G\alpha olf$, as in an olfactory receptor.

In the present specification, the term "broadly tuned olfactory receptor" refers to an olfactory receptor polypeptide that is an olfactory receptor responding to a plurality of odorants having different odors, more specifically, an olfactory receptor responding to a plurality of odorants belonging to different structural classes. Examples of the structural classes of odorants include amines, thiols, alcohols, esters, ethers, aldehydes, cyclic alkanes, terpenes, vanillins, camphors, azines, musks, ketones, acids, benzenes, lactones, and sulfides. The broadly tuned olfactory receptor in the present specification is an olfactory receptor responding to preferably 38% or more, more preferably 77% or more, of structural class groups when the responses to preferably 9 or more groups, more preferably 11 or more groups, and further preferably 13 or more groups of the above-mentioned structural classes are investigated. The phrase "respond to some structural class group" related to the olfactory receptor means that the olfactory receptor responds to at least one, preferably three or more, and more preferably four or more odorants belonging to the structural class group.

In the present specification, "enhancement of odor" and "suppression of odor" by some substance refers to a phenomenon in which the susceptibility to odor in the presence of the substance in an individual becomes stronger and weaker, respectively, compared the susceptibility in the absence of the substance. The degree of strength of susceptibility to odor can be evaluated on the basis of the detection strength or detection threshold value (sensitivity) of the odor. For example, when the detection strength of a target odor increases or the detection threshold value decreases (or the detection sensitivity increases), it is judged that the susceptibility to the target odor is enhanced, that is, the target odor is enhanced. In addition, for example, when detection strength of a target odor decreases or the detection threshold value increases (or the detection sensitivity decreases), it is judged that the susceptibility to the target odor is suppressed, that is, the target odor is suppressed. Alternatively, the degree of strength of susceptibility to odor reflects the degree of strength of olfactory sensitivity. The degree of strength of olfactory sensitivity can be evaluated on the basis of the responsiveness of the olfactory receptor. That is, the enhancement and decrease in the responsiveness of the olfactory receptor to some odor represent respectively the enhancement and suppression in the olfactory sensitivity to the odor. Accordingly, the degree of strength of susceptibility to an odor can be evaluated on the basis of the responsiveness of the olfactory receptor to the odor.

In the present specification, a "substance which causes enhancement in olfactory sensitivity" (so-called olfactory sensitivity enhancer) is a substance which enhances the response of an olfactory receptor to a target odorant when the substance is used together with the target odorant (for example, simultaneous with or prior to the use of the target odorant), compared with the case of using the target odorant alone. Alternatively, a "substance which causes enhancement in olfactory sensitivity" is a substance which causes enhancement of an odor. In the present specification, a "substance which causes suppression in olfactory sensitivity" (so-called olfactory sensitivity inhibitor) is a substance which decreases the response of an olfactory receptor to a target odorant when the substance is used together with the target odorant, compared with the case of using the target odorant alone. Alternatively, a "substance which causes suppression in olfactory sensitivity" is a substance which causes suppression of an odor. The olfactory sensitivity enhancer and inhibitor may have their own odors.

In the present specification, a "substance which causes enhancement of an odor" (so-called odor enhancer) is a substance which enhances the odor of an odor-causing substance when the substance is used together with the odor-causing substance (for example, simultaneous with or prior to the use of the odor-causing substance), compared with the case of using the causative substance alone. In the present specification, a "substance which causes suppression of an odor" (so-called odor inhibitor) is a substance which suppresses the odor of an odor-causing substance when the substance is used together with the causative substance, compared with the case of using the causative substance alone. The odor enhancer and the odor inhibitor may have their own odors.

In the past, it has been proposed a hypothesis about the role of broadly tuned olfactory receptors that the receptors recognize the total concentration of odors to control the sensory intensity, not to transmit the quality of the odor (Non-Patent Literature 2). However, different multiple hypotheses have also been proposed in addition to this hypothesis, but these hypotheses have not been verified. Furthermore, since there are a large number, 400 or more, of olfactory receptors and broadly tuned olfactory receptors have wide ranges of odorant responsiveness, it is difficult to activate a broadly tuned olfactory receptor only and to evaluate the influence of the activation on the odor sensation. Accordingly, in the past, reliable results of verification of the role of broadly tuned olfactory receptors have not been obtained.

Paradoxically, the fact that the above hypothesis (4) of the Non-Patent Literature 2 is proposed means that it is unclear at present how the sensory intensity of odor is produced. If a broadly tuned olfactory receptor controls the intensity of an odor, not the quality, the control of the activity is useful as a technology for more clearly feeling a smell regardless of the type thereof.

The present inventors identified human broadly tuned olfactory receptors responding to various odorants belonging to different structural classes. The inventors further found substances each selectively activating one broadly tuned olfactory receptor only and succeeded for the first time in verifying the roles of broadly tuned olfactory receptors by constructing single-broadly tuned olfactory receptor response models using the substances. As a result, the present inventors found that the odors of various odorants can be felt keenly or more strongly by enhancing the activities of broadly tuned olfactory receptors.

The present invention provides a method for selecting a substance which causes enhancement or suppression in olfactory sensitivity using the response of a broadly tuned olfactory receptor as an indicator. In addition, the present invention provides a method for selecting a substance which causes enhancement or suppression in odor sensation regardless of the type of odor using the response of a broadly tuned olfactory receptor as an indicator.

According to the present invention, a substance which can control olfactory sensitivity or enhance or suppress an odor can be efficiently selected based on the activating action on a broadly tuned olfactory receptor.

The present inventors revealed that "enhancement of an odor" and "suppression of an odor" occur by enhancement and decrease of the activity of a broadly tuned olfactory receptor. The olfactory sensitivity of an individual is enhanced or suppressed by activation or suppression of the broadly tuned olfactory receptor. That is, when olfactory cells respond to an odor-causing substance, a broadly tuned olfactory receptor is activated or suppressed prior to or simultaneous with the response to enhance or suppress the olfactory sensitivity of the individual or to cause an increase or decrease in the strength or sensitivity of detection of the odor recognized by the individual. Accordingly, a substance (for example, a receptor agonist) that enhances the activity of a broadly tuned olfactory receptor can be used for enhancement of the olfactory sensitivity or the odor in the present invention, and a substance (for example, a receptor antagonist) that decreases the activity of a broadly tuned olfactory receptor can be used for suppression in olfactory sensitivity or the odor in the present invention.

Accordingly, the present invention provides a method for selecting a substance which causes enhancement or suppression of an odor, the method comprising:

measuring a response of a broadly tuned olfactory receptor in the presence of a test substance; and
selecting a test substance which enhances or suppresses the response of the olfactory receptor as a substance which causes enhancement or suppression of an odor.

The present invention also provides a method for selecting a substance which causes enhancement or suppression in olfactory sensitivity, the method comprising:

measuring a response of a broadly tuned olfactory receptor in the presence of a test substance; and
selecting a test substance which enhances or suppresses the response of the olfactory receptor as a substance which causes enhancement or suppression in olfactory sensitivity.

The method of the present invention can be performed in vivo or ex vivo.

In the method of the present invention, first, at least one broadly tuned olfactory receptor is prepared. The broadly tuned olfactory receptor to be used in the method of the present invention is preferably a mammal-derived olfactory receptor. Examples of the mammal-derived olfactory receptor include olfactory receptors of primates, such as humans, chimpanzees, and monkeys; and rodents, such as mice and rats; preferred examples are human, mouse, and rat olfactory receptors; and more preferred examples are human olfactory receptors. Information on human, mouse, and rat olfactory receptors can be obtained from the GenBank [www.ncbi.nlm.nih.gov/genbank]. The broadly tuned olfactory receptor to be used in the method of the present invention may be an olfactory receptor derived from a single species of animal or may be a combination of olfactory receptors derived from two or more species of animal.

The broadly tuned olfactory receptor to be used in the method of the present invention can be obtained by searching a group of olfactory receptor polypeptides for a polypeptide satisfying the above-described definition of a "broadly tuned olfactory receptor" for identification of the polypeptide. For example, broadly tuned olfactory receptors are investigated for the responsiveness to various odorants as in Example 1 described below, and among them, an olfactory receptor satisfying the above-described definition of a "broadly tuned olfactory receptors" is selected and identified. The procedure of the search for a broadly tuned olfactory receptor will now be described in detail.

The olfactory receptor polypeptides to be supplied for searching a broadly tuned olfactory receptor can be used in an arbitrary form as long as the responsiveness to odorants is not lost. For example, the olfactory receptor can be used in the form of, for example, a tissue or cell naturally expressing the olfactory receptor, such as olfactory organ or olfactory cell isolated from a biological body, or a culture thereof; olfactory cell membrane supporting the olfactory receptor; a recombinant cell genetically engineered to express the olfactory receptor or a culture thereof; membrane of the recombinant cell having the olfactory receptor; or artificial lipid bilayer membrane having the olfactory receptor.

In a preferred aspect, the olfactory receptor polypeptides to be supplied for searching a broadly tuned olfactory receptor can be cells naturally expressing the olfactory receptor, such as mammalian olfactory cells, recombinant cells genetically engineered to express the olfactory receptor, or a culture thereof. Preferred examples thereof include recombinant human cells genetically engineered to express a human olfactory receptor.

The recombinant cell can be produced by transforming a cell with a vector incorporated with a gene encoding the olfactory receptor polypeptide. Alternatively, the olfactory receptor polypeptide can also be expressed by directly introducing a transcriptional product of the gene into a cell. Preferably, in order to promote the cellular membrane expression of an olfactory receptor polypeptide, a gene encoding a receptor-transporting protein (RTP) is introduced into a cell together with a gene encoding the olfactory receptor polypeptide. Preferably, a gene encoding RTP1S is introduced into a cell together with a gene encoding the olfactory receptor polypeptide. Examples of RTP1S include human RTP1S. Human RTP1S is a protein registered in the GenBank as GI:50234917.

Various odorants are added to the olfactory receptor polypeptide, and the responses to the respective substances are measured. This measurement may be performed by an arbitrary method known in the art as a method for measuring the response of an olfactory receptor, for example, intracellular cAMP level measurement. For example, it is known that an olfactory receptor activated by an odor molecule activates adenylate cyclase by conjugating with intracellular $G\alpha s$ to increase the amount of intracellular cAMP. Accordingly, the response of an olfactory receptor polypeptide to the causative substance can be measured using the intracellular cAMP level after the addition of the causative substance as an indicator. Examples of the method for measuring the cAMP level include an ELISA and a reporter gene assay. Another method for measuring the response of an olfactory receptor polypeptide is, for example, calcium imaging. Further another method is, for example, measurement by an electrophysiologic technique. In the electrophysiologic measurement, for example, a cell (e.g., Xenopus oocyte) coexpressing an olfactory receptor polypeptide and another ion channel is produced, and the activity of the ion channel on the cell is measured by, for example, a patch-clamp technique or a double electrode voltage-clamp technique, to measure the response of the olfactory receptor polypeptide.

As the odorants to be used for searching a broadly tuned olfactory receptor, a plurality of types of odorants having different odors are used. Preferably, multiple odorants in different structural classes are used. More preferably, a set of odorants including odorants belonging to 9 or more groups, further preferably 11 or more groups, and still more preferably 13 or more groups of 17 structural classes consisting of amines, thiols, alcohols, esters, ethers, aldehydes, cyclic alkanes, terpenes, vanillins, camphors, azines, musks, ketones, acids, benzenes, lactones, and sulfides is used. The set of odorants includes at least one, preferably three or more, and more preferably four or more odorants in each group of the above-described structural classes. However, one odorant is preferably added to an olfactory receptor to be supplied for searching in each response measurement.

Subsequently, the broadly tuned olfactory receptor is identified based on the measured response. The responsiveness can be evaluated by comparing the response of the olfactory receptor polypeptide (test group) to which the odorant is added with that in a control group. Examples of the control group include the olfactory receptor polypeptide mixed with the odorant with different concentrations, the olfactory receptor polypeptide not mixed with the odorant, the olfactory receptor polypeptide mixed with a control substance, the olfactory receptor polypeptide before the addition of the odorant, and a cell not expressing the olfactory receptor polypeptide.

When the response in a test group is enhanced compared with that in a control group, it is judged that the olfactory receptor polypeptide responds to the odorant. For example, if the response in a test group is enhanced to preferably 120% or more, more preferably 150% or more, and further preferably 200% or more of that in a control group, it is judged that the olfactory receptor polypeptide responds to the odorant. Alternatively, if the response in a test group is statistically significantly enhanced compared with that in a control group, it is judged that the olfactory receptor polypeptide responds to the odorant.

The odorant with different concentrations may be added to an olfactory receptor polypeptide, according to need, and the responses may be measured by the same procedure. If the response increases depending on the concentration of the odorant, it can be verified that the olfactory receptor polypeptide has responsiveness to the odorant.

Based on the response measurement above, an olfactory receptor polypeptide satisfying the definition of the "broadly tuned olfactory receptors" described above is selected. Preferably, when the responses thereof to 9 or more groups, more preferably 11 or more groups, and still more preferably 13 or more groups of 17 structural classes consisting of amines, thiols, alcohols, esters, ethers, aldehydes, cyclic alkanes, terpenes, vanillins, camphors, azines, musks, ketones, acids, benzenes, lactones, and sulfides (each group consists of at least one, preferably three or more, and more preferably four or more odorants) are investigated, an olfactory receptor polypeptide responding to preferably 38% or more and more preferably 77% or more of the investigated group is identified as a broadly tuned olfactory receptor.

The broadly tuned olfactory receptor identified by the procedure above can be used in the method of the present invention. The broadly tuned olfactory receptor used in the method of the present invention is, for example, at least one selected from the group consisting of human olfactory receptors OR2W1 (olfactory receptor family 2 subfamily W member 1), OR1A1 (olfactory receptor family 1 subfamily A member 1), and OR10A6 (olfactory receptor family 10 subfamily A member 6) and polypeptides having a function equivalent to those of the human olfactory receptors. Information on the nucleotide sequences and amino acid sequences of human olfactory receptors OR2W1, OR1A1, and OR10A6 is all registered in the NCBI (National Center for Biotechnology Information; [www.ncbi.nlm.nih.gov]). In the present specification, OR2W1, OR1A1, and OR10A6 refer to polypeptides having amino acid sequences represented by SEQ ID Nos: 1, 2, and 3, respectively.

Examples of the polypeptide having a function equivalent to that of OR2W1, OR1A1, or OR10A6 include olfactory receptor polypeptides consisting of an amino acid sequence having an identity of at least 80% to the amino acid sequence represented by SEQ ID NO: 1, 2, or 3 and responding to a wide range of odorants satisfying the definition of the "broadly tuned olfactory receptors" as in OR2W1, OR1A1, and OR10A6.

Preferably, the polypeptide having a function equivalent to that of OR2W1 is an olfactory receptor polypeptide that includes an amino acid sequence having an identity of at least 80% to the amino acid sequence represented by SEQ ID NO: 1 and activated by being bound to an odor molecule belonging to any of alcohols, aldehydes, esters, ketones, acids, terpenes, vanillins, benzenes, and lactones;

Preferably, the polypeptide having a function equivalent to that of OR1A1 is an olfactory receptor polypeptide that includes an amino acid sequence having an identity of at least 80% to the amino acid sequence represented by SEQ ID NO: 2 and activated by being bound to an odor molecule belonging to any of alcohols, aldehydes, esters, ketones, acids, terpenes, vanillins, benzenes, musks, and lactones; and

Preferably, the polypeptide having a function equivalent to that of OR10A6 is an olfactory receptor polypeptide that includes an amino acid sequence having an identity of at least 80% to the amino acid sequence represented by SEQ ID NO: 3 and is activated by binding of an odor molecule belonging to any of alcohols, aldehydes, esters, ketones, acids, terpenes, benzenes, cyclic alkanes, and lactones therto.

Other examples of the polypeptide having a function equivalent to that of human olfactory receptor OR2W1, OR1A1, or OR10A6 include mouse and rat orthologs of these human olfactory receptors, such as Olfr263 (another name: Olfr42, MOR256-31, mouse) and Olr1662 (rat) for OR2W1, Olfr43 (mouse) and Olr1513 and Olr1514 (rat) for OR1A1, and Olfr519 (mouse) and Olfatory Receptor 281 and Olfactory Receptor 10A3-Like (rat) for OR10A6.

In the method of the present invention, OR2W1, OR1A1, and OR10A6 and polypeptides having a function equivalent to those of the human olfactory receptors may be used alone or may be used in combination of two or more thereof. Preferably, at least one selected from the group consisting of OR2W1 and polypeptides having a function equivalent to that of OR2W1 is used.

In the method of the present invention, the broadly tuned olfactory receptors can be used in an arbitrary form as long as the broadly tuned responsiveness is not lost. For example, the broadly tuned olfactory receptor can be used in the form of, for example, a tissue or cell naturally expressing the olfactory receptor, such as olfactory organ or olfactory cell isolated from a biological body, or a culture thereof; olfactory cell membrane supporting the olfactory receptor; a recombinant cell genetically engineered to express the olfactory receptor or a culture thereof; membrane of the recombinant cell having the olfactory receptor; or artificial lipid bilayer membrane having the olfactory receptor. These forms are all included in the range of the broadly tuned olfactory receptors to be used in the present invention.

In a preferred aspect, the broadly tuned olfactory receptor to be used in the method of the present invention can be cells naturally expressing the broadly tuned olfactory receptor, such as mammalian olfactory cells, recombinant cells genetically engineered to express the broadly tuned olfactory receptor, or a culture thereof. Preferred examples thereof include recombinant human cells genetically engineered to express a human broadly tuned olfactory receptor.

The recombinant cell can be produced by transforming a cell with a vector incorporated with a gene encoding the broadly tuned olfactory receptor or by directly introducing a transcriptional product of the gene into a cell. Preferably, in order to promote the cellular membrane expression of an olfactory receptor, a gene encoding an RTP (preferably RTP1S such as human RTP1S) is introduced into a cell together with a gene encoding the olfactory receptor.

In the method of the present invention, first, the response of the broadly tuned olfactory receptor in the presence of a test substance is measured. Preferably, a test substance is added to the broadly tuned olfactory receptor, and the response of the olfactory receptor is then measured.

The test substance to be used in the method of the present invention may be any substance that is desired to be used as a substance which causes enhancement or suppression in olfactory sensitivity or an odor. The test substance may be a naturally occurring substance, an artificially synthesized substance by, for example, a chemical or biological method, or a compound or may be a composition or a mixture. The test substance need not be an odorant as long as the test substance can be applied to an olfactory receptor. Preferably, examples of the test substance do not include the following substances: fecal odors (for example, skatole and indole); body odor causative substances (for example, nonanoic acid, hexanoic acid, and isovaleric acid); butyl acrylate; putrid and urine odor causative substances (for example, 2-methoxy-4-vinylphenol and p-cresol); armpit odor causative substances (for example, 3-mercapto-3-methylhexanol and 3-methyl-2-hexanoic acid); muguet essence; trans-2-hexenal and cis-3-hexenol; and OR2W1 agonists and antagonists mentioned in Patent Literatures 2 to 13 (the entire contents of which are incorporated as reference).

A test substance can be added to an olfactory receptor by, for example, a method by adding a test substance to a medium for culturing cells expressing the olfactory receptor or a method by directly dropping, dispersing, or spraying a test substance to the olfactory receptor, but the method is not particularly limited.

The response of a broadly tuned olfactory receptor can be measured by an arbitrary method known in the art, for example, by measurement of an intracellular cAMP level by, for example, an ELISA or a reporter gene assay, calcium imaging, or an electrophysiologic technique, such as a patch-clamp technique and a double electrode voltage-clamp technique.

Subsequently, based on the measured response, the action of the test substance on the response of the broadly tuned olfactory receptor is evaluated. A test substance which enhances the response of a broadly tuned olfactory receptor is selected as a substance which causes enhancement in olfactory sensitivity or enhancement of an odor (so-called olfactory sensitivity enhancer or odor enhancer). In contrast, a test substance which decreases the response of the broadly tuned olfactory receptor is selected as a substance which causes suppression in olfactory sensitivity or suppression of an odor (so-called olfactory sensitivity inhibitor or odor inhibitor).

The action of a test substance on the response of a broadly tuned olfactory receptor can be evaluated by comparing the response of the broadly tuned olfactory receptor in the presence of the test substance (test group) with the response of the broadly tuned olfactory receptor in the absence of the test substance (control group). Examples of the control group include the olfactory receptor mixed with the test substance at different concentrations, the olfactory receptor not mixed with the test substance, the olfactory receptor mixed with a control substance, the olfactory receptor before the addition of the test substance, and a cell not expressing the olfactory receptor. Alternatively, the control group may be, for example, another olfactory receptor having no or low responsiveness to the test substance.

When the test substance is desired to be used as a substance which causes suppression in olfactory sensitivity or an odor, it is desirable to add a substance activating the broadly tuned olfactory receptor to both the test group and the control group, together with the test substance. Examples of the substance activating the receptor when the broadly tuned olfactory receptor is OR2W1 include fecal odors (for example, skatole and indole); body odor causative substances (for example, nonanoic acid, hexanoic acid, and isovaleric acid); butyl acrylate; putrid and urine odor causative substances (for example, 2-methoxy-4-vinylphenol and p-cresol); armpit odor causative substances (for example, 3-mercapto-3-methylhexanol and 3-methyl-2-hexanoic acid); muguet essence; trans-2-hexenal and cis-3-hexenol; and OR2W1 agonists mentioned in Patent Literatures 2 to 13 (the entire contents of which are incorporated as reference).

If the response in a test group is enhanced compared with the response in the control group, the test substance can be selected as a substance which enhances the response of the broadly tuned olfactory receptor. In contrast, if the response in a test group is decreased than the response in the control group, the test substance can be selected as a substance which decreases the response of the broadly tuned olfactory receptor. For example, the action of the test substance on the response of a broadly tuned olfactory receptor can be evaluated by comparing the responses of the broadly tuned olfactory receptor in a test substance addition group and a non-addition group, in a test substance addition group and a control substance addition group, before and after the addition of a test substance, or in a broadly tuned olfactory receptor expressing cell group and a non-expressing cell group. If the response of a broadly tuned olfactory receptor is induced or inhibited by the addition of a test substance, the test substance is selected as a substance which enhances or decreases the response of the broadly tuned olfactory receptor.

For example, if the response of a broadly tuned olfactory receptor in a test substance addition group is enhanced to preferably 120% or more, more preferably 150% or more, and further preferably 200% or more of that in a control group, the test substance can be selected as a substance which enhances the response of the broadly tuned olfactory receptor. Alternatively, if the response of a broadly tuned olfactory receptor in a test substance addition group is statistically significantly enhanced compared to that in a control group, the test substance can be selected as a substance which enhances the response of the broadly tuned olfactory receptor. The selected test substance can be obtained as an olfactory sensitivity enhancer or odor enhancer.

In addition, for example, if the response of a broadly tuned olfactory receptor in a test substance addition group is decreased to preferably 90% or less, more preferably 70% or less, and further preferably 50% or less of that in a control group, the test substance can be selected as a substance which decreases the response of the broadly tuned olfactory receptor. Alternatively, if the response of a broadly tuned olfactory receptor in a test substance addition group is statistically significantly decreased compared to that in a control group, the test substance can be selected as a substance which decreases the response of the broadly tuned olfactory receptor. The selected test substance can be obtained as an olfactory sensitivity inhibitor or odor inhibitor.

The action of enhancing or suppressing the olfactory sensitivity or odor of the test substance selected by the method of the present invention may be further sensorily evaluated, according to need. That it, in an embodiment of the method of the present invention, the test substance selected by the above-described procedure is obtained as a candidate substance of the substance which causes enhancement or suppression in olfactory sensitivity or an odor. Subsequently, the action of enhancing or suppressing the olfactory sensitivity or odor of the candidate substance is sensorily evaluated. For example, the detection strength or detection sensitivity to an odor is sensorily evaluated in the presence or absence of the candidate substance. If the detection strength or detection sensitivity to an odor is enhanced in the presence of the candidate substance, the candidate substance is selected as an olfactory sensitivity enhancer or odor enhancer (may be collectively referred to as "enhancer of the present invention"). In contrast, if the strength or sensitivity of an odor is decreased in the presence of the candidate substance, the candidate substance is selected as an olfactory sensitivity inhibitor or odor inhibitor (may be collectively referred to as "inhibitor of the present invention") . The odor to be used for the sensory evaluation may be a single odor or two or more odors of different types.

The type of the odor to be used in the sensory evaluation may be any type. For example, it is preferable to use an odor for which the candidate substance is intended to be used in order to enhance or suppress the intensity of the odor. For example, when the detection strength or detection sensitivity to a target odor that is desired to be enhanced or suppressed is enhanced or decreased, the candidate substance can be selected as an olfactory sensitivity or odor enhancer or inhibitor for the target odor. Alternatively, when the detection strength or detection sensitivity to odors of two or more different types is enhanced or decreased in the presence of a candidate substance, the candidate substance can be selected as a substance which causes enhancement or suppression in olfactory sensitivity to various odors or the odors (so-called "versatile" olfactory sensitivity or odor enhancer or inhibitor).

The enhancer or inhibitor of the present invention selected by the method of the present invention is a substance which can enhance or suppress a target odor by controlling the activity of a broadly tuned olfactory receptor. More specifically, the response of a broadly tuned olfactory receptor is enhanced or suppressed in the presence of the enhancer or inhibitor of the present invention, and thereby the detection strength or detection sensitivity to a target odor is increased or decreased. Consequently, the target odor is enhanced or suppressed. Accordingly, the enhancer or inhibitor of the present invention has an action of enhancing or suppressing an odor smelled at the time of application thereof or later. When the enhancer or inhibitor of the present invention is a so-called "versatile" olfactory sensitivity or odor enhancer or inhibitor, the enhancer or inhibitor works as a substance which causes enhancement or suppression of various odors.

An embodiment of using an enhancer or inhibitor of the present invention obtained by a method of the present invention is as follows: First, an enhancer or inhibitor of the present invention to a target odor is applied, prior to or simultaneous with exposure to the target odor, to the olfactory receptor of a subject who desires enhancement or suppression of the target odor. When the enhancer is applied, the susceptibility of the subject to the target odor is increased through activation of a broadly tuned olfactory receptor. As a result, the subject exposed to the target odor smells the target odor more strongly or detects the odor at a lower concentration (higher sensitivity). In contrast, when the inhibitor is applied, the susceptibility of the subject to the target odor is decreased through inhibition of the activity of a broadly tuned olfactory receptor. As a result, when the subject is exposed to the target odor, the subject smells the target odor more weakly or detects the odor at a higher concentration (lower sensitivity).

Examples of application of an enhancer of the present invention obtained by a method of the present invention include addition to city gas or propane gas for warning leakage of the gas; addition to a substance containing a fragrance agent (for example, cosmetics, cleaning agents, external preparations such as deodorant, laundry detergents, softeners, and other daily necessities, medicines, food, linens, sanitary products, and clothing) for obtaining clear feeling of the smell from the product even with a small amount of the fragrance agent; and application to a product having a target odor or an environment causing a target odor for obtaining clear feeling of the odor. Examples of application of an inhibitor of the present invention obtained by a method of the present invention include application to the space where suppression of offensive odors is desired, such as a toilet, a bathroom, a ward, a nursing home, or a residence including a kitchen, a bedroom, and a closet; addition to a product (for example, external preparations such as an air refresher and a deodorant, or a detergent, laundry detergent, or softener for deodorization) for odor suppression; addition to a product of which odor is desired to be suppressed (for example, disposable diapers, sanitary products, undergarment, underwear, linens, and other clothing, fabric or textiles, medicines, and food); and application to a production line of a product having a target odor or an environment causing a target odor for suppressing the odor. However, the examples of application of the enhancer or inhibitor of the present invention are not limited thereto.

As exemplary embodiments of the present invention, the following substances, manufacturing methods, uses, methods and the like are further disclosed in the present specification, but the present invention is not limited to these embodiments.

[1] A method for selecting a substance which causes enhancement or suppression of an odor, the method comprising:

measuring a response of a broadly tuned olfactory receptor in the presence of a test substance; and
selecting a test substance which enhances or decreases the response of the olfactory receptor as a substance which causes enhancement or suppression of an odor.

[2] The method according to aspect [1], wherein, preferably, the enhancement of an odor is an increase in the detection strength or detection sensitivity to an odor, and the suppression of an odor is a decrease in the detection strength or detection sensitivity to an odor.
[3] The method according to aspect [1] or [2], wherein, preferably, a test substance which enhances the response of the olfactory receptor is selected as a substance which causes enhancement of an odor.
[4] The method according to aspect [1] or [2], wherein, preferably, a test substance which decreases the response of the olfactory receptor is selected as a substance which causes suppression of an odor.
[5] A method for selecting a substance which causes enhancement or suppression in olfactory sensitivity, the method comprising:

measuring a response of a broadly tuned olfactory receptor in the presence of a test substance; and
selecting a test substance which enhances or decreases the response of the olfactory receptor as a substance which causes enhancement or suppression in olfactory sensitivity.

[6] The method according to aspect [5], wherein, preferably, the enhancement in olfactory sensitivity is an increase in the detection strength or detection sensitivity to an odor, and the suppression in olfactory sensitivity is a decrease in the detection strength or detection sensitivity to an odor.
[7] The method according to aspect [5] or [6], wherein, preferably, a test substance which enhances the response of the olfactory receptor is selected as a substance which causes enhancement in olfactory sensitivity.
[8] The method according to aspect [5] or [6], wherein, preferably, a test substance which decreases the response of the olfactory receptor is selected as a substance which causes suppression in olfactory sensitivity.
[9] The method according to any one of aspects [1] to [8], wherein the broadly tuned olfactory receptor is preferably an olfactory receptor responding to 38% or more of structural class groups of odorants, and the structural class groups of odorants are nine or more groups selected from the group consisting of amines, thiols, alcohols, esters, ethers, aldehydes, cyclic alkanes, terpenes, vanillins, camphors, azines, musks, ketones, acids, benzenes, lactones, and sulfides.
[10] The method according to any one of aspects [1] to [9], wherein the broadly tuned olfactory receptor is preferably a human, mouse, or rat-derived broadly tuned olfactory receptor;
more preferably at least one selected from the group consisting of OR2W1, OR1A1, and OR10A6 and polypeptides having a function equivalent to those of the human olfactory receptors; and

further preferably at least one selected from the group consisting of OR2W1 and polypeptides having a function equivalent to that of OR2W1.

[11] The method according to aspect [10], wherein, preferably,

OR2W1 is a polypeptide consisting of the amino acid sequence represented by SEQ ID NO: 1;
OR1A1 is a polypeptide consisting of the amino acid sequence represented by SEQ ID NO: 2;
OR10A6 is a polypeptide consisting of the amino acid sequence represented by SEQ ID NO: 3;

the polypeptide having a function equivalent to that of OR2W1 is an olfactory receptor polypeptide consisting of an amino acid sequence having an identity of at least 80% to the amino acid sequence represented by SEQ ID NO: 1 and activated by being bound to an odor molecule belonging to any of alcohols, aldehydes, esters, ketones, acids, terpenes, vanillins, benzenes, and lactones;

the polypeptide having a function equivalent to that of OR1A1 is an olfactory receptor polypeptide consisting of an amino acid sequence having an identity of at least 80% to the amino acid sequence represented by SEQ ID NO: 2 and activated by being bound to an odor molecule belonging to any of alcohols, aldehydes, esters, ketones, acids, terpenes, vanillins, benzenes, musks, and lactones; and

the polypeptide having a function equivalent to that of OR10A6 is an olfactory receptor polypeptide consisting of an amino acid sequence having an identity of at least 80% to the amino acid sequence represented by SEQ ID NO: 3 and activated by being bound to an odor molecule belonging to any of alcohols, aldehydes, esters, ketones, acids, terpenes, benzenes, cyclic alkanes, and lactones.

[12] The method according to aspect [11], wherein the identity of at least 80% is preferably an identity of 90% or more, more preferably an identity of 95% or more, and further preferably an identity of 99% or more.

[13] The method according to any one of aspects [1] to [12], preferably further comprising measuring the response of the broadly tuned olfactory receptor in the absence of the test substance.

[14] The method according to aspect [13], wherein preferably a test substance is selected as a substance which causes enhancement of an odor in the presence of the test substance to 120% or more of the response in the absence of the test substance.

[15] The method according to aspect [13], wherein preferably a test substance is selected as a substance which increase the response of the olfactory receptor in the presence of the test substance to 120% or more of the response in the absence of the test substance is selected as a substance which causes enhancement in olfactory sensitivity.

[16] The method according to aspect [13], wherein preferably a test substance is selected as a substance which causes enhancement of an odor in the presence of the test substance is increased statistically significantly manner compared to the response in the absence of the test substance.

[17] The method according to aspect [13], wherein preferably a test substance as a substance which causes enhancement in olfactory sensitivity in the presence of the test substance is increased in statistically significantly manner compared to the response thereof in the absence of the test substance.

[18] The method according to aspect [13], wherein preferably a test substance which decreases the response of the olfactory receptor in the presence of the test substance to 90% or less of the response in the absence of the test substance is selected as a substance which causes suppression of an odor.

[19] The method according to aspect [13], wherein preferably a test substance which decreases the response of the olfactory receptor in the presence of the test substance to 90% or less of the response in the absence of the test substance is selected as a substance which causes suppression in olfactory sensitivity.

[20] The method according to aspect [13], wherein preferably is selected as a substance which causes suppression of an odor in the presence of the test substance is decreased in statistically significantly manner compared to the response in the absence of the test substance.

[21] The method according to aspect [13], wherein preferably a test substance which statistically significantly decreases the response of the olfactory receptor in the presence of the test substance compared to the response in the absence of the test substance is selected as a substance which causes suppression in olfactory sensitivity.

[22] The method according to any one of aspects [1] to [21], wherein the broadly tuned olfactory receptor is preferably expressed on a recombinant cell genetically engineered to express the olfactory receptor.

[23] The method according to any one of aspects [1] to [22], wherein the response of the olfactory receptor is preferably measured by measurement of an intracellular cAMP level by an ELISA or a reporter gene assay, calcium imaging, or an electrophysiologic technique.

[24] The method according to any one of aspects [1] to [23], preferably further comprising sensorily evaluating the odor-enhancing or suppressing action of the selected test substance.

[25] The method according to any one of aspects [1] to [23], preferably further comprising sensorily evaluating the olfactory sensitivity-enhancing or suppressing action of the selected test substance.

Examples

The present invention will now be more specifically described by examples.

Reference Example

1) Test substance

Phenylethyl alcohol (PEA) (2-phenylethanol: Sigma-Aldrich Co. LLC)
Hexylcinnamaldehyde (HCA) (2-(phenylmethylidene)octanal) (Kao Corporation)
Hydroxycitronellal (HC) (7-hydroxy-3,7-dimethyloctanal: HC) (BASF)
L-Arginine (FUJIFILM Wako Pure Chemical Corporation)
L-Histidine (FUJIFILM Wako Pure Chemical Corporation)

2) Cloning of human olfactory receptor gene

Based on the sequence information registered in GenBank, genes encoding 427 human olfactory receptors including variants were each cloned. Each gene was cloned by PCR using human genomic DNA female (G1521: Promega) as the template. Each gene amplified by PCR was recombined downstream of a Flag-Rho tag sequence on a pME18S vector.

3) Construction of pME18S-human RTP1S vector

A gene encoding human RTP1S was inserted into the EcoRI/XhoI site of a pME18S vector.

4) Production of olfactory receptor expressing cell

427 HEK293 cells differently expressing one of 427 human olfactory receptors were produced. Reaction solutions having compositions shown in Table 1 were prepared and were left to stand in a clean bench for 15 minutes. The reaction solutions were added to each well of a multi-well plate (384 or 96-well plate, BioCoat), and HEK293 cells were then seeded. In the 384-well plate, 4.4 $\mu$L of a reaction solution and 40 $\mu$L of HEK293 cells ($20 \times 10^4$ cells/cm$^2$) were added. In the 96-well plate, 10 $\mu$L of a reaction solution and 90 $\mu$L of HEK293 cells ($3 \times 10^5$ cells/cm$^2$) were added. The cells were cultured in an incubator maintained at 37°C and 5% CO$_2$ for 24 hours. As a control, cells not expressing an olfactory receptor (mock) were prepared.

[Table 1]

| Reaction solution composition for 384-well plate (per well) | |
| --- | --- |
| DMEM (Nacalai tesque, INC.) | 2.2 $\mu$L |
| TE (Tris-EDTA) | 2.2 $\mu$L |
| Human olfactory receptor gene (inserted into pME18S vector, expressed with Flag-Rho tag at N-terminus) | 0.029 $\mu$g |
| pGL4.29 (fluc2P-CRE-hygro, Promega) | 0.022 $\mu$g |
| pGL4.75 (hRluc-CMV, Promega) | 0.0012 $\mu$g |
| pME18S-human RTP1S vector | 0.012 $\mu$g |
| lipofectamine 2000 (Invitrogen) or PEI-MAX (Polyscience) | 0.16 $\mu$L |
| Reaction, solution composition for 96-well plate (per well) | |
| DMEM (Nacalai tesque, INC.) | 10 $\mu$L |
| Human olfactory receptor gene (inserted into pME18S vector, expressed with Flag-Rho tag at N-terminus) | 0.075 $\mu$g |
| pGL4.29 (fluc2P-CRE-hygro, Promega) | 0.03 $\mu$g |
| pGL4.75 (hRluc-CMV, Promega) | 0.03 $\mu$g |

(continued)

| Reaction, solution composition for 96-well plate (per well) | |
|---|---|
| pME18S-human RTP1S vector | 0.03 μg |
| PEI-MAX (Polyscience) | 0.41 μL |

5) Luciferase assay of olfactory receptor

The olfactory receptor expressed in HEK293 cells interact with endogenous cellular Gαs to activate adenylate cyclase and thereby increases the intracellular cAMP level. The measurement of the response of cells to an odorant, test substance, or antagonist in the present invention was performed by a luciferase reporter gene assay monitoring the increase in the intracellular cAMP level as a light emission value derived from a firefly luciferase gene (fluc2P-CRE-hygro). A Renilla luciferase gene fused to downstream of a CMV promoter (hRluc-CMV) was simultaneously transfected and was used as an internal standard for calibrating the error in gene transfer efficiency or the number of cells. The luciferase activity was measured from 2.5 to 4 hours after stimulation with an odorant or a test substance with a Dual-Glo™ luciferase assay system (Promega), following user's manual thereof. The value fLuc/hRluc was calculated by dividing the light emission value derived from firefly luciferase by the light emission value derived from Renilla luciferase. "Fold increase" as an indicator of the responsiveness of an olfactory receptor to stimulation with a test substance, and "odor response" (%) as an indicator of the activity level of an antagonist to the olfactory receptor, were calculated according to the following formula:

$$\text{Fold increase} = X/Y$$

X: fLuc/hRluc, induced by stimulation with a test substance alone, and
Y: fLuc/hRluc, without stimulation with a test substance.

$$\text{Odor response (\%)} = (Z'-Y')/(X'-Y') \times 100$$

X': fLuc/hRluc, induced by stimulation with an odorant alone,
Y': fLuc/hRluc, without stimulation with an odorant, and
Z': fLuc/hRluc, induced by co-stimulation with an odorant and an antagonist.

Example 1 Evaluation of ligand selectivity of human olfactory receptor

Response selectivity of olfactory receptors to various odorants with different structural classes was investigated. In this Example, 33 compounds selected from 11 groups (alcohols, aldehydes, esters, ketones, acids, terpenes, vanillins, benzenes, musks, cyclic alkanes, and lactones, each group including three or more odorants) based on similarity in the chemical structures were used as odorants.

DMEM solutions (Nacalai tesque, INC.) of each odorant were prepared at concentration (30 to 3000 μM) as high as possible taking the solubility and cytotoxicity of the odorant into accounts. The medium was removed from the culture of cells expressing an olfactory receptor prepared according to Reference Example 2) to 4), 30 μL of the odorant solution was added to each well, and luciferase assay was performed by the same procedure as in Reference Example 5). The cells were cultured for from 2.5 to 4 hours in a $CO_2$ incubator to obtain sufficient expression of the luciferase gene in the cells, and the expression level was measured using the light emission quantity as an indicator.

The results are shown in Figure 1. Figure 1 shows odorant groups to which the cells expressing human olfactory receptor OR1A1, OR2W1, or OR10A6 responded. The group in which an olfactory receptor responded to all three substances is designated as "ACTIVE" (filled circle). The group in which an olfactory receptor did not respond to one or more substances is designated as "INACTIVE" (-). As shown in Figure 1, OR1A1 responded to about 90% (10 groups of 11 odorant groups), and OR2W1 and OR10A6 responded to about 80% (9 groups of 11 odorant groups) of the investigated odorant groups. This result demonstrated that these olfactory receptors are broadly tuned olfactory receptors which can recognize various groups of odorants.

Example 2 Broadly tuned olfactory receptor selective ligand

L-Arginine and l-histidine were used as test substances. These test substances were each dissolved in Ringer's solution (140 mM NaCl, 5 mM KCl, 1 mM $MgCl_2$, 2 mM $CaCl_2$, 10 mM HEPES, 5 mM glucose, pH 7.4 (NaOH)) to prepare test substance solutions. OR2W1 expressing cells were subjected to luciferase assay using the test substance solutions according to the procedure in Reference Example 2) to 5). The results are shown in Figure 2. OR2W1 responded to l-arginine concentration-dependently (Figure 2, upper middle). In contrast, OR2W1 expressing cells did not respond to 1-histidine (Figure 2, bottom middle).

Furthermore, the responses of 427 olfactory receptors to l-arginine and l-histidine (10 mM) were investigated according to Reference Example 2) to 5). The results demonstrated that no olfactory receptors other than OR2W1 responded to l-arginine (Figure 2, upper right). All the investigated olfactory receptors did not respond to l-histidine (Figure 2, bottom right). It was accordingly demonstrated that 1-arginine is a substance selectively activating OR2W1.

Example 3 Influence on olfaction by nasal administration of l-arginine

1) Effect of odor on detection threshold value (detection sensitivity)

The action of 1-arginine, which is an OR2W1 selective ligand, on olfaction was evaluated by sensory examination. Three samples, saline (Otsuka Pharmaceutical Co., Ltd.), saline aqueous solution containing 10 mM l-arginine, and saline aqueous solution containing 10 mM 1-histidine, were prepared and filled into nasal sprays (KT110-102, AS ONE Corporation). As an odorant, phenylethyl alcohol (PEA), which is widely used in sensory examination as a substance and does not activate the trigeminal nerve system and gives a rose odor, was selected. PEA aqueous solutions with 11 different concentrations from 0.001 to 100 $\mu$M were prepared as test solutions, and 3 mL of each of the solutions was respectively added to 20-mL glass vials (Maruemu Corporation).

The experiment was blinded to both an examiner and an examinee. The examiner set the spray spout in the nasal cavity of the examinee as deep as possible and sprayed once. The amount of liquid ejected thereby was about 100 to 200 $\mu$L. Before the spraying and one minute after the spraying, two vials each containing an aqueous solution only and one vial containing a test solution, three vials in total, were presented to the examinee, and the examinee was questioned about which vial contained the odor. When the vial of the test solution was correctly indicated, a retest was performed using a test solution with one step lower concentration. When the answer was incorrect, a retest was performed using a test solution with one step higher concentration. The concentration that gave a correct answer three consecutive times after answering an incorrect answer was determined as the detection threshold value. The threshold test performed again after the spraying was started from a concentration ten times the threshold value concentration before the spraying. The threshold test was performed by nasal administration of any of saline, arginine, and histidine. After an interval of 2.5 hours or more, a threshold test was performed by nasal administration of another sample. Before and after the nasal administration of a sample, detection threshold values of an examinee to PEA were measured. The rate of change in the threshold value was determined by calculating (threshold value concentration before sample nasal administration)/(threshold value concentration after sample nasal administration).

As a result, in an examinee after nasal administration of l-arginine, the PEA detection threshold value was decreased in statistically significant manner (Figure 3). Such an effect was not observed in nasal administration of saline and l-histidine which do not activate OR2W1. These results revealed that l-arginine raises the human detection threshold value (detection sensitivity) of a PEA odor and suggested that OR2W1, which is only one activated by l-arginine, brings the effect.

2) Effect of odor on detection strength

Whether the odor of an odorant was enhanced or not by simultaneous exposure to the odorant and 1-arginine was investigated by comparing with exposure to the odorant alone. PEA or hexyl cinnamic aldehyde (HCA) was used as the odorant. The odorant was added at a final concentration of 0.1 mM to a saline aqueous solution with l-arginine (10 mM) and a saline aqueous solution with 1-histidine (10 mM) as a control. The resulting solutions were sprayed to an examinee for inhaling using an ultrasonic nebulizer (NE-U07: OMRON Corporation, atomization particle diameter: 1 to 8 $\mu$m (80% of total volume particle diameter distribution)) with a spray amount of about 170 $\mu$L (for about 20 seconds) at most, and odor intensity was evaluated.

The odor intensity was evaluated according to the method of Green, et al, (Chemical senses, 1996, 21: 323-34) with the labeled magnitude scale (LMS) shown below. That is, assuming the full scale length was 100%, labels were set to the following positions: Barely detectable: 1.4%, Weak: 6.1%, Moderate: 17.2%, Strong: 35.4%, Very strong: 53.3%, Strongest imaginable: 100%.

[Formula 1]

Labeled Magnitude Scale (LMS)

- Strongest imaginable

- Very Strong

- Strong

- Moderate

- Weak
- Barely Detectable

As a result, the odor intensities of both odorants, PEA and HCA, were enhanced in statistically significant manner by using together with 1-arginine (Figure 4). In contrast, in the use together with l-histidine, the effect of enhancing the odor intensity was not observed in both odorants. These results revealed that l-arginine raises the detection strength of human for an odor and suggested that OR2W1, which is only one activated by 1-arginine, brings the effect.

3) Effect of odor on quality

If a broadly tuned olfactory receptor controls only the strength of an odor, not the quality, activation with OR2W1 alone is expected not to turn the odor quality (specific odor). Accordingly, whether or not human odor sensation occurs was investigated by activating, among about 400 olfactory receptors, only of OR2W1 by 1-arginine. Since l-arginine is not volatile, 1-arginine dissolved in saline was administered in the nasal cavity of five examinees by nasal spray. As a result, nasal administration of l-arginine did not cause odor sensation. In nasal administration of another substance by the same procedure, odor sensation occurred in three of five examinees, which demonstrated that the substance nasally administered by this method reached the olfactory epithelium. These results suggest that the activation of OR2W1 is not involved in occurrence of odor quality (specific odor). The experimental results further support the hypothesis that broadly tuned olfactory receptors do not generate the odor quality (specific odor) (see Non-Patent Literature 2).

Example 4 Action of PEA and HCA on OR2W1

The response of OR2W1 expressing cells to PEA and HCA was investigated. The results show that OR2W1 responded to PEA, but did not respond to HCA (Figure 5). This demonstrated that the action of 1-arginine shown in Figure 4 is not limited to enhancement of the odors of odorants to which OR2W1 responds, such as PEA, and can be applied to enhancement of the odors to which OR2W1 does not respond, such as HCA.

Example 5 Influence of OR2W1 activation on odor intensity

1) As shown in Figure 1, OR2W1 responded to 80% of 11 odorant groups. In contrast, responsiveness of OR2W1 to only subtle fragrance agents having weak smells was investigated, and the results were that clear responses were observed in only less than 10% of 68 fragrance agents. These results suggested exist of a relationship between the strength of the OR2W1 activation ability of a fragrance agent and odor intensity.

2) The action of enhancing the odors of other fragrance agents by fragrance agents having different actions of activating OR2W1 was investigated. A subtle fragrance agent (compound A) showed no OR2W1 activating action and a subtle fragrance agent (compound B) showed the highest activating action in the above 1) were used as test substances, and the odor enhancing effect of PEA or hydroxycitronellal (HC) was investigated. Two cotton balls were prepared. One of them was soaked with a test substance (compound A or B), and the other was soaked with an odorant (PEA or HC). The cotton balls were put in respective glass vials to prepare samples. The odorant alone, the odorant + compound A, the odorant + compound B, and the odorant alone were presented, in this order, to five examinees to evaluate the odor intensities of odorant samples. In addition, the odor intensity of each of compounds A and B alone was evaluated. The evaluation of odor intensity was performed by the same procedure as that in Example 3. The results are shown in Figure 6.

3) Furthermore, 10 OR2W1 antagonist compounds were investigated for the skatole odor suppressing action and skatole receptor activity suppressing action. OR2W1, OR5K1, OR5P3, and OR8H1 are known as skatole receptors (Japanese Patent No. 5593271), and among them, the possibility of controlling skatole odor intensity by OR2W1 was verified. The odor response (%) in the presence of the antagonist compound was measured by the same procedure as in Reference Example 2) to 5).

The skatole odor suppressing action of an antagonist compound was investigated by the following procedure. A cotton ball was put in a glass bottle (Hakuyo Glass No. 11, capacity: 110 mL), and 20 $\mu$L of skatole as an offensive odor diluted $10^5$ times with propylene glycol and 20 $\mu$L of an antagonist compound were each added dropwise to the cotton ball. The glass bottle was left to stand at room temperature overnight for sufficiently vaporizing the odor molecules in the glass bottle, and the skatole odor was then evaluated by odor sensory examination. In the sensory examination, the odor intensity of skatole odor alone was defined as 5, and the offensive odor intensity of a mixture with an antagonist compound was evaluated by 20 grades from 0 to 10 (in units of 0.5). The smaller value means the weaker skatole odor. The sensory examination was performed by 4 to 8 panelists, and the average of the results was determined.

The results are shown in Figure 7. The horizontal axis in each graph of Figure 7 represents the antagonist compound. The vertical axis of the left graph represents OR2W1 activity (odor response (%)) on skatole in the presence of each antagonist compound, and a higher odor response (%) means a lower antagonist activity of the antagonist compound. The vertical axis of the right graph represents the strength of the skatole odor suppressing action of the antagonist compound (the average of sensory evaluation by 4 to 8 panelists). The compounds suppressed the skatole recognition by OR2W1 in the receptor assay all suppressed the skatole odor in the sensory examination. This result suggests that the activity of OR2W1 strongly contributes to recognition of the intensity of such an odor.

The results of the above 1) to 3), based on the results of Examples 3 and 4, suggest that the olfactory receptor OR2W1 is an olfactory receptor which transmits information on the odor intensity, not the odor quality, of various odorants.

## Claims

1. A method for selecting a substance which causes enhancement or suppression of an odor, the method comprising:

   measuring a response of a broadly tuned olfactory receptor in the presence of a test substance; and
   selecting a test substance which enhances or decreases the response of the olfactory receptor as a substance which causes enhancement or suppression of an odor.

2. A method for selecting a substance which causes enhancement or suppression in olfactory sensitivity, the method comprising:

   measuring a response of a broadly tuned olfactory receptor in the presence of a test substance; and
   selecting a test substance which enhances or decreases the response of the olfactory receptor as a substance which causes enhancement or suppression in olfactory sensitivity.

3. The method according to claim 1 or 2, wherein the broadly tuned olfactory receptor is at least one selected from the group consisting of human olfactory receptors OR2W1, OR1A1, and OR10A6 and polypeptides having a function equivalent to those of the human olfactory receptors.

4. The method according to claim 1 or 2, wherein the broadly tuned olfactory receptor is at least one selected from the group consisting of human olfactory receptor OR2W1 and polypeptides having a function equivalent to that of the human olfactory receptor.

5. The method according to claim 3 or 4, wherein

   OR2W1, is a polypeptide consisting of the amino acid sequence represented by SEQ ID NO: 1;
   OR1A1 is a polypeptide consisting of the amino acid sequence represented by SEQ ID NO: 2; and

OR10A6 is a polypeptide consisting of the amino acid sequence represented by SEQ ID NO: 3.

6. The method according to any one of claims 3 to 5, wherein
the polypeptide having a function equivalent to that of OR2W1 is an olfactory receptor polypeptide consisting of an amino acid sequence having an identity of at least 80% to the amino acid sequence represented by SEQ ID NO: 1 and activated by being bound to an odor molecule belonging to any of alcohols, aldehydes, esters, ketones, acids, terpenes, vanillins, benzenes, and lactones;
the polypeptide having a function equivalent to that of OR1A1 is an olfactory receptor polypeptide consisting of an amino acid sequence having an identity of at least 80% to the amino acid sequence represented by SEQ ID NO: 2 and activated by being bound to an odor molecule belonging to any of alcohols, aldehydes, esters, ketones, acids, terpenes, vanillins, benzenes, musks, and lactones; and
the polypeptide having a function equivalent to that of OR10A6 is an olfactory receptor polypeptide consisting of an amino acid sequence having an identity of at least 80% to the amino acid sequence represented by SEQ ID NO: 3 and activated by being bound to an odor molecule belonging to any of alcohols, aldehydes, esters, ketones, acids, terpenes, benzenes, cyclic alkanes, and lactones.

7. The method according to claim 6, wherein the identity of at least 80% is an identity of 90% or more.

8. The method according to claim 6, wherein the identity of at least 80% is an identity of 95% or more.

9. The method according to claim 6, wherein the identity of at least 80% is an identity of 99% or more.

10. The method according to any one of claims 1 to 9, further comprising:
measuring a response of the broadly tuned olfactory receptor in the absence of the test substance.

11. The method according to claim 10, wherein the test substance is selected as a substance response of an olfactory receptor in the presence of the test substance is increased in statistically significantly manner or for 120% or more compared to the response thereof in the absence of the test substance.

12. The method according to claim 10, wherein the test substance is selected as a substance response of an olfactory receptor in the presence of the test substance is increased in statistically significantly manner or for 90% or more compared to the response thereof in the absence of the test substance.

13. The method according to claim 10, wherein the test substance is selected as a substance which causes enhancement in olfactory sensitivity in the presence of the test substance is increased in statistically significantly manner or for 90% or more compared to the response thereof in the absence of the test substance.

14. The method according to claim 10, wherein the test substance is selected as a substance which causes suppression in olfactory sensitivity in the presence of the test substance is increased in statistically significantly manner or for 120% or more compared to the response thereof in the absence of the test substance.

15. The method according to any one of claims 1 to 14, wherein
the broadly tuned olfactory receptor is expressed on a recombinant cell genetically engineered to express the olfactory receptor.

16. The method according to any one of claims 1 to 15, wherein
the response of an olfactory receptor is measured by an intracellular cAMP level measurement by an ELISA or a reporter gene assay, calcium imaging, or an electrophysiologic technique.

17. The method according to any one of claims 1 to 16, further comprising:
sensorily evaluating the odor-enhancing or suppressing action of the selected test substance.

[Figure 1]

[Figure 2]

[Figure 3]

[Figure 4]

[Figure 5]

[Figure 6]

EP 3 578 976 A1

[Figure 7]

26

| | INTERNATIONAL SEARCH REPORT | International application No. |
|---|---|---|
| | | PCT/JP2018/001504 |

**A. CLASSIFICATION OF SUBJECT MATTER**
Int.Cl. G01N33/50(2006.01)i, C12Q1/68(2018.01)i, G01N33/15(2006.01)i, G01N33/497(2006.01)i, G01N33/53(2006.01)i, G01N33/84(2006.01)i, C07K14/705(2006.01)n
According to International Patent Classification (IPC) or to both national classification and IPC

**B. FIELDS SEARCHED**

Minimum documentation searched (classification system followed by classification symbols)
Int.Cl. G01N33/50, C12Q1/68, G01N33/15, G01N33/497, G01N33/53, G01N33/84, C07K14/705

Documentation searched other than minimum documentation to the extent that such documents are included in the fields searched
Published examined utility model applications of Japan 1922-1996
Published unexamined utility model applications of Japan 1971-2018
Registered utility model specifications of Japan 1996-2018
Published registered utility model applications of Japan 1994-2018

Electronic data base consulted during the international search (name of data base and, where practicable, search terms used)
JSTPlus/JMEDPlus/JST7580(JDreamIII), PubMed

**C. DOCUMENTS CONSIDERED TO BE RELEVANT**

| Category* | Citation of document, with indication, where appropriate, of the relevant passages | Relevant to claim No. |
|---|---|---|
| X | JP 2012-249614 A (KAO CORPORATION) 20 December 2012, claims, examples 4, 5 & US 2014/0186864 A1, claims, examples 4, 5 & WO 2012/169644 A1 & EP 2718718 A1 | 1-17 |
| X | JP 2012-50411 A (KAO CORPORATION) 15 March 2012, claims, examples 4, 5 & US 2013/0216492 A1, claims, examples 4, 5 & WO 2012/029922 A1 & EP 2612923 A1 | 1-17 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.02.2018 | 27.02.2018 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2018/001504

C (Continuation).   DOCUMENTS CONSIDERED TO BE RELEVANT

| Category* | Citation of document, with indication, where appropriate, of the relevant passages | Relevant to claim No. |
|---|---|---|
| A | YU, Y. et al., Responsiveness of G protein-coupled odorant receptors is partially attributed to the activation mechanism, Proc Natl Acad Sci USA, 01 December 2015, vol. 112, no. 48, 14966-14971 | 1-17 |
| A | CHARLIER L. et al., How broadly tuned olfactory receptors equally recognize their agonists, human OR1G1 as a test case, Cell Mol Life Sci, 2012, 69, 4205-4213 | 1-17 |
| A | US 2015/0260707 A1 (SYMRISE AG) 17 September 2015, entire text & EP 2884280 A1 & CN 104726551 A | 1-17 |
| A | US 2007/0020210 A1 (MONELL CHEMICAL SENSES CENTER) 25 January 2007, entire text & WO 2003/061609 A1 & EP 1474095 A1 | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015020158 A
- JP 5798382 B
- JP 5593271 B
- JP 5982044 B
- JP 5646255 B
- JP 5697383 B
- WO 2012029922 A

- JP 2015091802 A
- JP 2015202075 A
- JP 2015211667 A
- JP 2015202077 A
- JP 5520173 B
- JP 2016008955 A

**Non-patent literature cited in the description**

- **NARA, K. et al.** *J. Neurosci.,* 2011, vol. 31, 9179-91
- **YU, Y. et al.** *Proc. Natl. Acad. Sci. USA,* 2015, vol. 112, 14966-71

- *Science,* 1985, vol. 227, 1435-41
- **GREEN et al.** *Chemical senses,* 1996, vol. 21, 323-34

SEQUENCE LISTING

<110>  Kao Corporation

<120>  Method of selecting olfaction controller

<130>  KS1547

<150>  JP 2017-017380
<151>  2017-02-02

<160>  3

<170>  PatentIn version 3.5

<210>  1
<211>  320
<212>  PRT
<213>  Homo sapiens

<220>
<223>  Human OR2W1


<400>  1

Met Asp Gln Ser Asn Tyr Ser Ser Leu His Gly Phe Ile Leu Leu Gly
1                   5                   10                  15


Phe Ser Asn His Pro Lys Met Glu Met Ile Leu Ser Gly Val Val Ala
                20                  25                  30


Ile Phe Tyr Leu Ile Thr Leu Val Gly Asn Thr Ala Ile Ile Leu Ala
            35                  40                  45


Ser Leu Leu Asp Ser Gln Leu His Thr Pro Met Tyr Phe Phe Leu Arg
        50                  55                  60


Asn Leu Ser Phe Leu Asp Leu Cys Phe Thr Thr Ser Ile Ile Pro Gln
65                  70                  75                  80


Met Leu Val Asn Leu Trp Gly Pro Asp Lys Thr Ile Ser Tyr Val Gly
                85                  90                  95


Cys Ile Ile Gln Leu Tyr Val Tyr Met Trp Leu Gly Ser Val Glu Cys
            100                 105                 110


Leu Leu Leu Ala Val Met Ser Tyr Asp Arg Phe Thr Ala Ile Cys Lys
        115                 120                 125


Pro Leu His Tyr Phe Val Val Met Asn Pro His Leu Cys Leu Lys Met
        130                 135                 140


Ile Ile Met Ile Trp Ser Ile Ser Leu Ala Asn Ser Val Val Leu Cys

```
                145                     150                    155                        160


                Thr Leu Thr Leu Asn Leu Pro Thr Cys Gly Asn Asn Ile Leu Asp His
                                165                    170                        175


                Phe Leu Cys Glu Leu Pro Ala Leu Val Lys Ile Ala Cys Val Asp Thr
                                180                    185                        190


                Thr Thr Val Glu Met Ser Val Phe Ala Leu Gly Ile Ile Ile Val Leu
                                195                    200                        205


                Thr Pro Leu Ile Leu Ile Leu Ile Ser Tyr Gly Tyr Ile Ala Lys Ala
                        210                    215                    220


                Val Leu Arg Thr Lys Ser Lys Ala Ser Gln Arg Lys Ala Met Asn Thr
                225                    230                    235                        240


                Cys Gly Ser His Leu Thr Val Val Ser Met Phe Tyr Gly Thr Ile Ile
                                245                    250                        255


                Tyr Met Tyr Leu Gln Pro Gly Asn Arg Ala Ser Lys Asp Gln Gly Lys
                                260                    265                    270


                Phe Leu Thr Leu Phe Tyr Thr Val Ile Thr Pro Ser Leu Asn Pro Leu
                                275                    280                    285


                Ile Tyr Thr Leu Arg Asn Lys Asp Met Lys Asp Ala Leu Lys Lys Leu
                                290                    295                    300


                Met Arg Phe His His Lys Ser Thr Lys Ile Lys Arg Asn Cys Lys Ser
                305                    310                    315                        320


                <210>   2
                <211>   309
                <212>   PRT
                <213>   Homo sapiens

                <220>
                <223>   Human OR1A1

                <400>   2

                Met Arg Glu Asn Asn Gln Ser Ser Thr Leu Glu Phe Ile Leu Leu Gly
                1                   5                   10                         15


                Val Thr Gly Gln Gln Glu Gln Glu Asp Phe Phe Tyr Ile Leu Phe Leu
                                20                     25                         30


                Phe Ile Tyr Pro Ile Thr Leu Ile Gly Asn Leu Leu Ile Val Leu Ala
                        35                     40                         45
```

```
Ile Cys Ser Asp Val Arg Leu His Asn Pro Met Tyr Phe Leu Leu Ala
    50                  55                  60

Asn Leu Ser Leu Val Asp Ile Phe Phe Ser Ser Val Thr Ile Pro Lys
65                  70                  75                  80

Met Leu Ala Asn His Leu Leu Gly Ser Lys Ser Ile Ser Phe Gly Gly
                85                  90                  95

Cys Leu Thr Gln Met Tyr Phe Met Ile Ala Leu Gly Asn Thr Asp Ser
            100                 105                 110

Tyr Ile Leu Ala Ala Met Ala Tyr Asp Arg Ala Val Ala Ile Ser Arg
            115                 120                 125

Pro Leu His Tyr Thr Thr Ile Met Ser Pro Arg Ser Cys Ile Trp Leu
        130                 135                 140

Ile Ala Gly Ser Trp Val Ile Gly Asn Ala Asn Ala Leu Pro His Thr
145                 150                 155                 160

Leu Leu Thr Ala Ser Leu Ser Phe Cys Gly Asn Gln Glu Val Ala Asn
                165                 170                 175

Phe Tyr Cys Asp Ile Thr Pro Leu Leu Lys Leu Ser Cys Ser Asp Ile
            180                 185                 190

His Phe His Val Lys Met Met Tyr Leu Gly Val Gly Ile Phe Ser Val
        195                 200                 205

Pro Leu Leu Cys Ile Ile Val Ser Tyr Ile Arg Val Phe Ser Thr Val
    210                 215                 220

Phe Gln Val Pro Ser Thr Lys Gly Val Leu Lys Ala Phe Ser Thr Cys
225                 230                 235                 240

Gly Ser His Leu Thr Val Val Ser Leu Tyr Tyr Gly Thr Val Met Gly
                245                 250                 255

Thr Tyr Phe Arg Pro Leu Thr Asn Tyr Ser Leu Lys Asp Ala Val Ile
            260                 265                 270

Thr Val Met Tyr Thr Ala Val Thr Pro Met Leu Asn Pro Phe Ile Tyr
        275                 280                 285

Ser Leu Arg Asn Arg Asp Met Lys Ala Ala Leu Arg Lys Leu Phe Asn
```

```
                  290                    295                      300


        Lys Arg Ile Ser Ser
        305


        <210>  3
        <211>  314
        <212>  PRT
        <213>  Homo sapiens

        <220>
        <223>  Human OR10A6

        <400>  3

        Met Glu Arg Gln Asn Gln Ser Cys Val Val Glu Phe Ile Leu Leu Gly
        1                   5                   10                  15


        Phe Ser Asn Tyr Pro Glu Leu Gln Gly Gln Leu Phe Val Ala Phe Leu
                    20                  25                  30


        Val Ile Tyr Leu Val Thr Leu Ile Gly Asn Ala Ile Ile Ile Val Ile
                    35                  40                  45


        Val Ser Leu Asp Gln Ser Leu His Val Pro Met Tyr Leu Phe Leu Leu
                    50                  55                  60


        Asn Leu Ser Val Val Asp Leu Ser Phe Ser Ala Val Ile Met Pro Glu
        65                  70                  75                  80


        Met Leu Val Val Leu Ser Thr Glu Lys Thr Thr Ile Ser Phe Gly Gly
                        85                  90                  95


        Cys Phe Ala Gln Met Tyr Phe Ile Leu Leu Phe Gly Gly Ala Glu Cys
                    100                 105                 110


        Phe Leu Leu Gly Ala Met Ala Tyr Asp Arg Phe Ala Ala Ile Cys His
                    115                 120                 125


        Pro Leu Asn Tyr Gln Met Ile Met Asn Lys Gly Val Phe Met Lys Leu
                    130                 135                 140


        Ile Ile Phe Ser Trp Ala Leu Gly Phe Met Leu Gly Thr Val Gln Thr
        145                 150                 155                 160


        Ser Trp Val Ser Ser Phe Pro Phe Cys Gly Leu Asn Glu Ile Asn His
                            165                 170                 175


        Ile Ser Cys Glu Thr Pro Ala Val Leu Glu Leu Ala Cys Ala Asp Thr
                    180                 185                 190
```

```
Phe Leu Phe Glu Ile Tyr Ala Phe Thr Gly Thr Phe Leu Ile Ile Leu
        195             200             205

Val Pro Phe Leu Leu Ile Leu Leu Ser Tyr Ile Arg Val Leu Phe Ala
    210             215             220

Ile Leu Lys Met Pro Ser Thr Thr Gly Arg Gln Lys Ala Phe Ser Thr
225             230             235             240

Cys Ala Ala His Leu Thr Ser Val Thr Leu Phe Tyr Gly Thr Ala Ser
            245             250             255

Met Thr Tyr Leu Gln Pro Lys Ser Gly Tyr Ser Pro Glu Thr Lys Lys
        260             265             270

Val Met Ser Leu Ser Tyr Ser Leu Leu Thr Pro Leu Leu Asn Leu Leu
        275             280             285

Ile Tyr Ser Leu Arg Asn Ser Glu Met Lys Arg Ala Leu Met Lys Leu
        290             295             300

Trp Arg Arg Arg Val Val Leu His Thr Ile
305             310
```